# EUROPEAN PATENT APPLICATION

(11) **EP 3 123 925 A1**
(43) Date of publication of application: **01.02.2017**
(21) Application number: 15770426.3
(22) Date of filing: 09.02.2015
(51) Int. Cl.: A61B 1/04, A61B 1/00

(54) **ENDOSCOPE SYSTEM, PROCESSOR DEVICE OF ENDOSCOPE SYSTEM, AND METHOD FOR OPERATING ENDOSCOPE SYSTEM**

(30) Priority: 28.03.2014 JP 2014069803
(71) Applicant: FUJIFILM Corporation, Tokyo 106-8620 (JP)
(72) Inventor: KOSHIBA Masaaki, Ashigarakami-gun Kanagawa 258-8538 (JP)
(74) Representative: Klunker . Schmitt-Nilson . Hirsch
(86) International application number: PCT/JP2015/053477
(87) International publication number: WO 2015/146318

(57) **Abstract**

There are provided an endoscope system, a processor apparatus for an endoscope system, and a method for operating an endoscope system with which the brightness and S/N of a normal observation image obtained in a special observation mode can be increased. In the special observation mode, second illumination light including different-absorption-wavelength light that has different absorption coefficients for oxygenated hemoglobin and reduced hemoglobin is emitted, a turn-off state is thereafter initiated, and signal reading is performed for some of a plurality of pixel rows in accordance with a skip read method. Subsequently, first illumination light, which is normal light, is emitted, a turn-off state is thereafter initiated, and signal reading is performed for all of the pixel rows in a column direction in accordance with a pixel addition read method. An image processor generates an oxygen saturation image on the basis of a second image capture signal read in accordance with the skip read method and a first image capture signal read in accordance with the pixel addition read method, and generates a normal observation image on the basis of the first image capture signal.

## Description

### BACKGROUND OF THE INVENTION

### 1. Field of the Invention

The present invention relates to an endoscope system, a processor apparatus for an endoscope system, and a method for operating an endoscope system having a special observation mode.

### 2. Description of the Related Art

In the field of medicine, diagnoses and so on using endoscope systems that include a light source apparatus, an endoscope, and a processor apparatus are widely made. Some endoscope systems have a special observation mode in which first illumination light and second illumination light having different spectral properties are emitted to an observation region in a living body and observation is performed.

In the special observation mode, the first illumination light and the second illumination light are alternately supplied to the endoscope from the light source apparatus and are emitted to the observation region from the distal end section of the endoscope. The first illumination light is white light (normal light), and the second illumination light is special light including light that has a high absorption coefficient for blood hemoglobin, for example. A normal observation image is generated by capturing an image of the observation region illuminated with the first illumination light, and a special observation image is generated by capturing an image of the observation region illuminated with the second illumination light.

In a conventional endoscope system, a CCD (Charge Coupled Device) image sensor is used as an image sensor of the endoscope. However, a CMOS (Complementary Metal-Oxide Semiconductor) image sensor has been increasingly used in recent years (see Japanese Unexamined Patent Application Publication No. 2010-68992). This is because a CMOS image sensor has lower power consumption than a CCD image sensor, and peripheral circuits, such as an ADC (analog-to-digital converter) circuit, can be formed on the same substrate on which an image capture unit is formed. The CMOS image sensor basically employs a rolling shutter method in which resetting and signal reading are performed for a plurality of pixel rows formed on the image capture unit one by one sequentially. The period from resetting to signal reading for each pixel row is the exposure period.

In the rolling shutter method, the exposure timing for each of the pixel rows shifts one by one sequentially. Therefore, when the illumination light is switched from the first illumination light to the second illumination light without interruption while the image sensor is driven in accordance with the rolling shutter method, the exposure periods of some pixel rows extend beyond the switching of the illumination light, and an image of light in which the first illumination light and the second illumination light mix is captured. Therefore, Japanese Unexamined Patent Application Publication No. 2010-68992 proposes a technique in which a turn-off period is provided upon switching of the illumination light and signal reading is performed during the turn-off period.

If a turn-off period is simply provided upon switching between the first illumination light and the second illumination light as described above, the frame rate of the image sensor decreases due to the provided turn-off period. Therefore, in Japanese Unexamined Patent Application Publication No. 2010-68992, the emission time (exposure time) of the first illumination light and that of the second illumination light are reduced, and the read time is reduced by decreasing the number of pixels of the image sensor for which signal reading is performed to thereby prevent the frame rate from decreasing.

However, in the endoscope system described in Japanese Unexamined Patent Application Publication No. 2010-68992, the first illumination light is continuously emitted and the image sensor is driven in accordance with the rolling shutter method in a normal observation mode while the exposure time is reduced so as to suppress a decrease in the frame rate in the special observation mode. Therefore, the brightness and S/N of a normal observation image obtained in the special observation mode are lower than those of a normal observation image obtained in the normal observation mode, which is a problem.

### SUMMARY OF THE INVENTION

An object of the present invention is to provide an endoscope system, a processor apparatus for an endoscope system, and a method for operating an endoscope system with which the brightness and S/N of a normal observation image obtained in the special observation mode can be increased.

In order to accomplish the above-described object, an endoscope system according to the present invention is an endoscope system including: an illumination unit that emits to a test body first illumination light and second illumination light having different spectral properties; an endoscope including an image sensor of CMOS type that captures an image of the test body illuminated by the illumination unit by using a plurality of pixel rows arranged in a column direction; a controller that causes the illumination unit and the image sensor to perform a first image capture method in which the second illumination light is emitted from the illumination unit, the illumination unit is thereafter turned off, signal reading is performed for pixel rows among the plurality of pixel rows in accordance with a skip read method, the first illumination light is emitted from the illumination unit, the illumination unit is thereafter turned off, and signal reading is performed for the plurality of pixel rows in the column direction in accordance with a pixel addition read method; and an image processor that generates a special observation image on the basis of a second image capture signal read in accordance with the skip read method and a first image capture signal read in accordance with the pixel addition read method, and generates a normal observation image on the basis of the first image capture signal.

Preferably, the controller controls the image sensor to reset only the pixel rows for which signal reading has been performed in accordance with the skip read method after signal reading in accordance with the skip read method has been performed.

Preferably, the image sensor includes a color filter based on a Bayer arrangement, in the skip read method, reading is performed sequentially in sets of two pixel rows adjacent to each other in the column direction while immediately succeeding two pixel rows are skipped, and in the pixel addition read method, reading is performed while addition for each set of two pixels on which color filters of an identical color are arranged is performed in the column direction.

In the image sensor, paired pixels adjacent to each other may selectively share a floating diffusion section, in the skip read method, only a signal charge accumulated in one of the paired pixels may be read via the floating diffusion section, and in the pixel addition read method, signal charges accumulated in the paired pixels may be added together by the floating diffusion section and read.

Preferably, the first illumination light or the second illumination light includes different-absorption-wavelength light that has different absorption coefficients for oxygenated hemoglobin and reduced hemoglobin, and the image processor generates an oxygen saturation image that includes information about an oxygen saturation level as the special observation image.

Preferably, the image processor generates the oxygen saturation image by calculating the oxygen saturation level on the basis of the first image capture signal and the second image capture signal and performing image processing on the normal observation image on the basis of the oxygen saturation level.

Preferably, the controller enables the illumination unit and the image sensor to perform a second image capture method in which the first illumination light is emitted from the illumination light, the illumination unit is thereafter turned off, signal reading is performed in accordance with the skip read method, the second illumination light is emitted from the illumination unit, the illumination unit is thereafter turned off, and signal reading is performed in accordance with the skip read method.

Preferably, the endoscope system further includes a brightness detection unit that detects brightness of the test body, and the controller causes the first image capture method to be performed in a case where the brightness has a value smaller than a certain value, and causes the second image capture method to be performed in a case where the brightness has a value equal to or larger than the certain value.

A processor apparatus for an endoscope system according to the present invention is a processor apparatus for an endoscope system, the endoscope system including an illumination unit that emits to a test body first illumination light and second illumination light having different spectral properties, and an endoscope including an image sensor of CMOS type that captures an image of the test body illuminated by the illumination unit by using a plurality of pixel rows arranged in a column direction, the processor apparatus including: a controller that causes the illumination unit and the image sensor to perform an image capture method in which the second illumination light is emitted from the illumination unit, the illumination unit is thereafter turned off, signal reading is performed for pixel rows among the plurality of pixel rows in accordance with a skip read method, the first illumination light is emitted from the illumination unit, the illumination unit is thereafter turned off, and signal reading is performed for the plurality of pixel rows in the column direction in accordance with a pixel addition read method; and an image processor that generates a special observation image on the basis of a second image capture signal read in accordance with the skip read method and a first image capture signal read in accordance with the pixel addition read method, and generates a normal observation image on the basis of the first image capture signal.

A method for operating an endoscope system according to the present invention is a method for operating an endoscope system, the endoscope system including an illumination unit that emits to a test body first illumination light and second illumination light having different spectral properties, and an endoscope including an image sensor of CMOS type that captures an image of the test body illuminated by the illumination unit by using a plurality of pixel rows arranged in a column direction, the method including the steps of: causing, by a controller, the illumination unit and the image sensor to perform an image capture method in which the second illumination light is emitted from the illumination unit, the illumination unit is thereafter turned off, signal reading is performed for pixel rows among the plurality of pixel rows in accordance with a skip read method, the first illumination light is emitted from the illumination unit, the illumination unit is thereafter turned off, and signal reading is performed for the plurality of pixel rows in the column direction in accordance with a pixel addition read method; and generating, by an image processor, a special observation image on the basis of a second image capture signal read in accordance with the skip read method and a first image capture signal read in accordance with the pixel addition read method, and generating a normal observation image on the basis of the first image capture signal.

According to the present invention, in the special observation mode, the second illumination light is emitted from the illumination unit, the illumination unit is thereafter turned off, signal reading is performed for pixel rows among the plurality of pixel rows in accordance with the skip read method, the first illumination light is emitted from the illumination unit, the illumination unit is thereafter turned off, signal reading is performed for the plurality of pixel rows in the column direction in accordance with the pixel addition read method, a special observation image is generated on the basis of a second image capture signal read in accordance with the skip read method and a first image capture signal read in accordance with the pixel addition read method, and a normal observation image is generated on the basis of the first image capture signal. Accordingly, it is possible to increase the brightness and S/N of the normal observation image obtained in the special observation mode.

### BRIEF DESCRIPTION OF THE DRAWINGS

Fig. 1 is an external view of an endoscope system;
Fig. 2 is a front view of a distal end section of an endoscope;
Fig. 3 is a block diagram illustrating an electrical configuration of the endoscope system;
Fig. 4 is a graph illustrating the intensity spectra of first and second illumination light;
Fig. 5 is a diagram illustrating an electrical configuration of an image sensor;
Fig. 6 is a circuit diagram illustrating a configuration of pixels;
Figs. 7A to 7C are diagrams for describing an operation of a column ADC circuit;
Fig. 8 is a diagram illustrating a configuration of a color filter array;
Fig. 9 is a diagram illustrating the spectral transmission properties of color filters;
Fig. 10 is a diagram for describing an image capture method in a normal observation mode;
Fig. 11 is a diagram for describing an image capture method in a special observation mode;
Fig. 12 is a block diagram illustrating a configuration of an image processor;
Fig. 13 is a graph illustrating correlations between signal ratios and oxygen saturation levels;
Fig. 14 is a graph illustrating the absorption coefficients of oxygenated hemoglobin and reduced hemoglobin;
Fig. 15 is a flowchart illustrating an operation of the endoscope system;
Fig. 16 is a diagram for describing an operation performed upon skip reading;
Fig. 17 is a diagram for describing an operation performed upon pixel addition reading;
Fig. 18 is a diagram illustrating a configuration of a CMOS image sensor based on an EXR arrangement scheme;
Fig. 19 is a circuit diagram illustrating a configuration of a pixel pair;
Fig. 20 is a diagram for describing driving timings in accordance with a second image capture method;
Fig. 21 is a flowchart for describing a method for switching between a first image capture method and the second image capture method; and
Fig. 22 is a diagram illustrating a configuration of a capsule endoscope.

### DESCRIPTION OF THE PREFERRED EMBODIMENTS

### First Embodiment

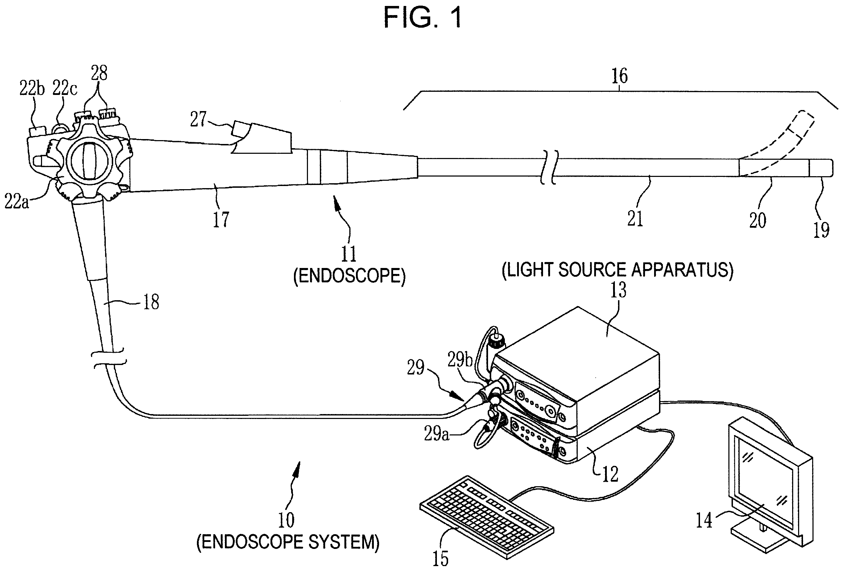

In Fig. 1, an endoscope system 10 includes an endoscope 11 that captures an image of an observation region (test body) in a living body, a processor apparatus 12 that generates a display image of the observation region on the basis of an image capture signal obtained as a result of image capture, a light source apparatus 13 that supplies illumination light for illuminating the observation region to the endoscope 11, and a monitor 14 that displays the display image. To the processor apparatus 12, the monitor 14 and an input unit 15, such as a keyboard or a mouse, are connected.

The endoscope 11 includes an insertion section 16 that is inserted into the alimentary canal of the living body, an operation section 17 that is provided to the base end portion of the insertion section 16, and a universal cord 18 that is used to connect the endoscope 11 with the processor apparatus 12 and the light source apparatus 13. The insertion section 16 is constituted by a distal end section 19, a bending section 20, and a flexible tube section 21, which are coupled to each other in this order from the distal end side.

The operation section 17 is provided with an angulation knob 22a, a mode switching switch 22b, and so on. The angulation knob 22a is used in an operation for bending the bending section 20. By operating the angulation knob 22a, the distal end section 19 can be turned in a desired direction.

The mode switching switch 22b is used in a switching operation between two types of observation modes, namely, a normal observation mode and a special observation mode. The normal observation mode is a mode in which a normal observation image obtained by capturing a full-color image of an observation target using white light is displayed on the monitor 14. The special observation mode is a mode in which the oxygen saturation level of blood hemoglobin of an observation target is calculated, and an oxygen saturation image obtained by performing image processing on a normal observation image on the basis of the oxygen saturation level is displayed on the monitor 14.

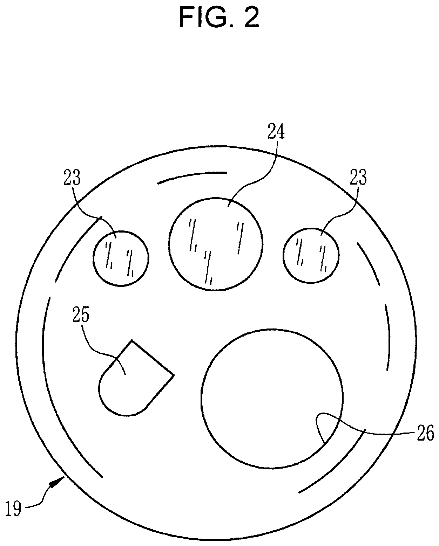

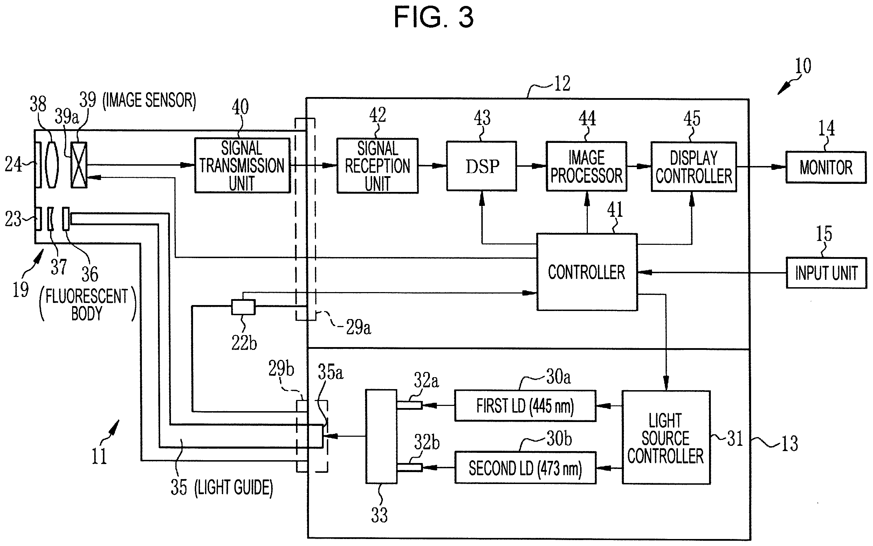

In Fig. 2, on the distal end face of the distal end section 19, illumination windows 23 through which illumination light is emitted to an observation region, an observation window 24 for taking in an image of the observation region, an air and water supply nozzle 25 for supplying air and water for cleaning the observation window 24, and a forceps outlet 26 through which medical tools, such as forceps and an electric knife, protrude for performing various treatments are provided. Behind the observation window 24, an image sensor 39 (see Fig. 3) is provided.

The bending section 20 is constituted by a plurality of bending pieces coupled to each other and bends in the up-down and right-left directions in response to an operation of the angulation knob 22a of the operation section 17. By bending the bending section 20, the distal end section 19 is turned in a desired direction. The flexible tube section 21 has flexibility and can be inserted into a winding canal, such as an esophagus and bowels. A signal cable used to transmit control signals for driving the image sensor 39 and image capture signals output from the image sensor 39, and light guides 35 (see Fig. 3) used to guide illumination light supplied from the light source apparatus 13 to the illumination windows 23 are inserted into and pass through the insertion section 16.

The operation section 17 is provided with a forceps inlet 27 through which medical tools are inserted, air and water supply buttons 28 that are operated in order to supply air and water through the air and water supply nozzle 25, a freeze button (not illustrated) used to capture a still image, and so on in addition to the angulation knob 22a and the mode switching switch 22b.

A communication cable extended from the insertion section 16 and the light guides 35 are inserted into and pass through the universal cord 18, and a connector 29 is attached to one end of the universal cord 18 on the side close to the processor apparatus 12 and the light source apparatus 13. The connector 29 is a combination-type connector constituted by a communication connector 29a and a light source connector 29b. The communication connector 29a and the light source connector 29b are detachably connected to the processor apparatus 12 and to the light source apparatus 13 respectively. At the communication connector 29a, one end of the communication cable is located. At the light source connector 29b, entrance ends 35a (see Fig. 3) of the light guides 35 are located.

In Fig. 3, the light source apparatus 13 includes a first laser diode (LD) 30a, a second LD 30b, a light source controller 31, a first optical fiber 32a, a second optical fiber 32b, and an optical coupler 33. The first LD 30a emits first blue laser light having a center wavelength of 445 nm. The second LD 30b emits second blue laser light having a center wavelength of 473 nm. The half width of the first and second blue laser light is about ±10 nm. As the first LD 30a and the second LD 30b, broad-area InGaN laser diodes, InGaNAs laser diodes, GaNAs laser diodes, and so on are used.

The light source controller 31 controls the first LD 30a and the second LD 30b to turn on and off the LDs individually. In the normal observation mode, the light source controller 31 turns on the first LD 30a. In the special observation mode, the light source controller 31 turns on the first LD 30a and the second LD 30b sequentially.

The first blue laser light emitted from the first LD 30a enters the first optical fiber 32a. The second blue laser light emitted from the second LD 30b enters the second optical fiber 32b. The first optical fiber 32a and the second optical fiber 32b are connected to the optical coupler 33. The optical coupler 33 unites the optical path of the first optical fiber 32a and that of the second optical fiber 32b and causes the first blue laser light and the second blue laser light to respectively enter the entrance ends 35a of the light guides 35 of the endoscope 11.

The endoscope 11 includes the light guides 35, a fluorescent body 36, an illumination optical system 37, an image capture optical system 38, the image sensor 39, and a signal transmission unit 40. One light guide 35 is provided for each of the illumination windows 23. As the light guides 35, multimode fibers can be used. For example, small-diameter fiber cables having a core diameter of 105 µm, a clad diameter of 125 µm, and a diameter including a protective layer that serves as an outer sheath of 0.3 to 0.5 mm can be used.

When the light source connector 29b is coupled to the light source apparatus 13, the entrance ends 35a of the respective light guides 35 located at the light source connector 29b face the emission end of the optical coupler 33. The fluorescent body 36 is located so as to face the emission ends of the respective light guides 35 positioned in the distal end section 19. The first blue laser light or the second blue laser light enters the fluorescent body 36 through a corresponding one of the light guides 35.

The fluorescent body 36 is formed by dispersing a plurality of types of fluorescent materials (YAG fluorescent materials or BAM (BaMgALS₁₀O₁₇) fluorescent materials, for example) in a binder and forming the fluorescent materials in a rectangular parallelepiped form. The fluorescent body 36 absorbs a portion of laser light (the first blue laser light or the second blue laser light) entering through a corresponding one of the light guides 35, is excited, and emits fluorescence having a wavelength band ranging from green to red. The portion of the laser light that enters the fluorescent body 36 passes through the fluorescent body 36 without being absorbed by the fluorescent body 36. As a result, the fluorescence and the portion of the laser light are emitted from the fluorescent body 36.

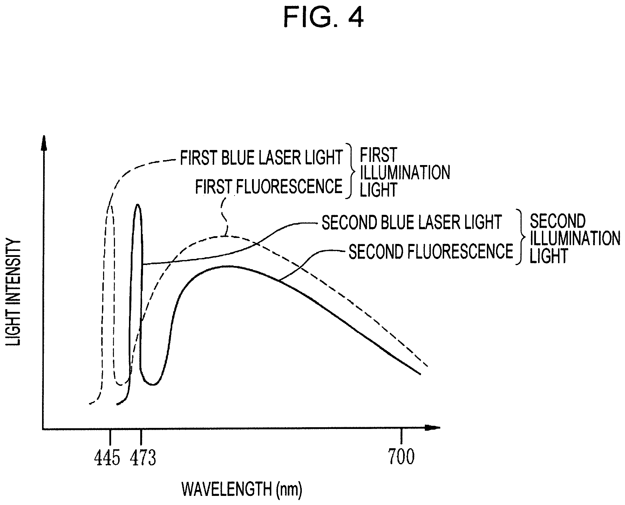

Specifically, in a case where the first LD 30a is turned on and the first blue laser light enters the fluorescent body 36, first illumination light having a spectrum illustrated in Fig. 4 is emitted from the fluorescent body 36. The first illumination light includes the first blue laser light and first fluorescence emitted from the fluorescent body 36 as a result of excitation in response to entry of the first blue laser light. In a case where the second LD 30b is turned on and the second blue laser light enters the fluorescent body 36, second illumination light having a spectrum illustrated in Fig. 4 is emitted from the fluorescent body 36. The second illumination light includes the second blue laser light and second fluorescence emitted from the fluorescent body 36 as a result of excitation in response to entry of the second blue laser light and has a spectral property different from that of the first illumination light. The spectral shapes of the first fluorescence and the second fluorescence are substantially the same. That is, the ratio between the intensity I₁(λ) of the first fluorescence and the intensity I₂(λ) of the second fluorescence at a wavelength λ is substantially constant.

The first illumination light and the second illumination light emitted from the fluorescent body 36 are condensed by the illumination optical system 37 and are emitted to an observation region in a living body via the illumination windows 23. Reflection light from the observation region enters the image capture optical system 38 via the observation window 24, and an image is formed on an image capture face 39a of the image sensor 39 by the image capture optical system 38. In this embodiment, the light source apparatus 13, the light guides 35, the fluorescent body 36, and the illumination optical system 37 correspond to an illumination unit described in the appended claims.

The image sensor 39 is of the CMOS type that captures an image of the reflection light from the observation region and outputs an image capture signal on the basis of an image capture control signal supplied from the processor apparatus 12.

The signal transmission unit 40 transmits the image capture signal obtained by the image sensor 39 to the processor apparatus 12 in accordance with a known low-voltage-operating signaling transmission method. When the above-described mode switching switch 22b provided to the endoscope 11 is operated, a mode switching operation signal is transmitted to the processor apparatus 12 from the mode switching switch 22b.

The processor apparatus 12 includes a controller 41, a signal reception unit 42, a digital signal processor (DSP) 43, an image processor 44, and a display controller 45. The controller 41 controls the components in the processor apparatus 12 and also controls the image sensor 39 of the endoscope 11 and the light source controller 31 of the light source apparatus 13.

The signal reception unit 42 receives the image capture signal transmitted from the signal transmission unit 40 of the endoscope 11. The DSP 43 performs known signal processing on the image capture signal received by the signal reception unit 42, such as a defect correction process, a gain correction process, a white balance process, gamma conversion, and a synchronization process.

The image processor 44 generates a normal observation image in the normal observation mode by performing a color conversion process, a color enhancement process, a structure enhancement process, and the like on an image capture signal that is obtained by the image sensor 39 capturing an image of reflection light from the observation region to which the first illumination light is emitted and that is subjected to signal processing performed by the DSP 43.

The image processor 44 generates an oxygen saturation image (special observation image) that includes information about the oxygen saturation level in the special observation mode by calculating the oxygen saturation level and a normal observation image on the basis of image capture signals that are obtained by the image sensor 39 capturing images of reflection light from the observation region to which the first illumination light and the second illumination light are emitted and that are subjected to signal processing performed by the DSP 43, and performing image processing on the normal observation image on the basis of the oxygen saturation level.

The display controller 45 converts the image generated by the image processor 44 into a signal in a display form and causes the monitor 14 to display the image.

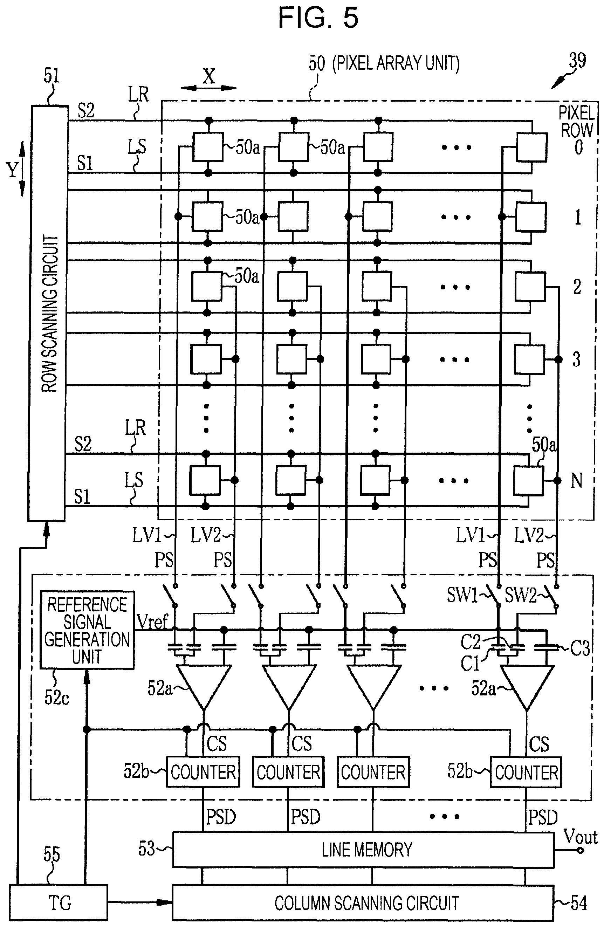

In Fig. 5, the image sensor 39 includes a pixel array unit 50, a row scanning circuit 51, a column ADC circuit 52 including a plurality of ADCs (analog-to-digital converters) that are arranged in a row direction, a line memory 53, a column scanning circuit 54, and a timing generator (TG) 55. The TG 55 generates timing signals on the basis of image capture control signals input from the controller 41 of the processor apparatus 12 and controls each component.

The pixel array unit 50 is constituted by a plurality of pixels 50a that are arranged in a 2D matrix in the row direction (X direction) and in a column direction (Y direction), and is provided on the image capture face 39a described above. In the pixel array unit 50, row selection lines LS and row reset lines LR are laid in the row direction, and first column signal lines LV1 and second column signal lines LV2 are laid in the column direction.

One row selection line LS and one row reset line LR are provided for each pixel row. One first column signal line LV1 and one second column signal line LV2 are provided for each pixel column. Here, the pixel row corresponds to the pixels 50a for one row that are arranged in the row direction, and the pixel column corresponds to the pixels 50a for one column that are arranged in the column direction.

The pixels 50a in one pixel row are connected in common to one set of a corresponding one of the row selection lines LS and a corresponding one of the row reset lines LR. Each of the pixels 50a is connected to a corresponding one of the first column signal lines LV1 or a corresponding one of the second column signal lines LV2. Specifically each pixel 50a in pixel rows 0, 1, 4, 5, 8, 9, ..., N-3, and N-2 (hereinafter referred to as a first pixel row group) among all of the pixel rows is connected to a corresponding one of the first column signal lines LV1. Each pixel 50a in the remaining pixel rows 2, 3, 6, 7, 10, 11, ..., N-1, and N (hereinafter referred to as a second pixel row group) is connected to a corresponding one of the second column signal lines LV2.

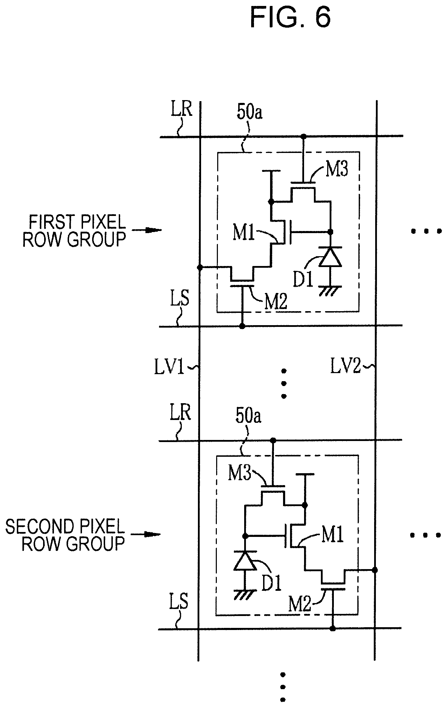

Each of the pixels 50a includes a photodiode D1, an amplifier transistor M1, a pixel selection transistor M2, and a reset transistor M3, as illustrated in Fig. 6. The photodiode D1 performs photoelectric conversion on incident light, generates a signal charge that corresponds to the amount of incident light, and accumulates the signal charge. The amplifier transistor M1 converts the signal charge accumulated by the photodiode D1 into a voltage value (pixel signal PS). The pixel selection transistor M2 is controlled by a corresponding one of the row selection lines LS and causes the pixel signal PS generated by the amplifier transistor M1 to be output to a corresponding one of the first column signal lines LV1 or a corresponding one of the second column signal lines LV2. The reset transistor M3 is controlled by a corresponding one of the row reset lines LR and releases into a power supply line (resets) the signal charge accumulated by the photodiode D 1.

The row scanning circuit 51 generates a row selection signal S1 and a reset signal S2 on the basis of timing signals input from the TG 55. The row scanning circuit 51 supplies the row selection signal S1 to any of the row selection lines LS upon a signal read operation to thereby cause the pixel signals PS of the pixels 50a connected to the row selection line LS to be output to the first column signal lines LV1 or to the second column signal lines LV2. The row scanning circuit 51 supplies the reset signal S2 to any of the row reset lines LR upon a reset operation to thereby reset the pixels 50a connected to the row reset line LR.

The column ADC circuit 52 includes comparators 52a, counters 52b, a reference signal generation unit 52c, first capacitors C1, second capacitors C2, third capacitors C3, first switches SW1, and second switches SW2. To the first input terminal of each of the comparators 52a, a corresponding one of the first capacitors C1 and a corresponding one of the second capacitors C2 are connected in parallel. To the second input terminal of each of the comparators 52a, a corresponding one of the third capacitors C3 is connected. To the output terminal of each of the comparators 52a, a corresponding one of the counters 52b is connected. For each pixel column, one set of a corresponding one of the first column signal lines LV1 and a corresponding one of the second column signal lines LV2 is provided, and the first column signal line LV1 and the second column signal line LV2 are connected to the first capacitor C1 and the second capacitor C2 of a corresponding one of the comparators 52a via a corresponding one of the first switches SW1 and a corresponding one of the second switches SW2 respectively.

The first switches SW1 and the second switches SW2 are controlled to be turned on and off on the basis of timing signals input from the TG 55, and either the first switches SW1 or the second switches SW2 are turned on or both the first switches SW1 and the second switches SW2 are turned on upon signal reading. Specifically, in a case where reading of pixel signals PS is performed for the first pixel row group, the first switches SW1 are turned on, and the pixel signals PS are input to the first capacitors C1. In a case where reading of pixel signals PS is performed for the second pixel row group, the second switches SW2 are turned on, and the pixel signals PS are input to the second capacitors C2. In a case of pixel addition reading described below, both the first switches SW1 and the second switches SW2 are turned on, and pixel signals PS read from the first pixel row group and those read from the second pixel row group are input to the first capacitors C1 and to the second capacitors C2 respectively.

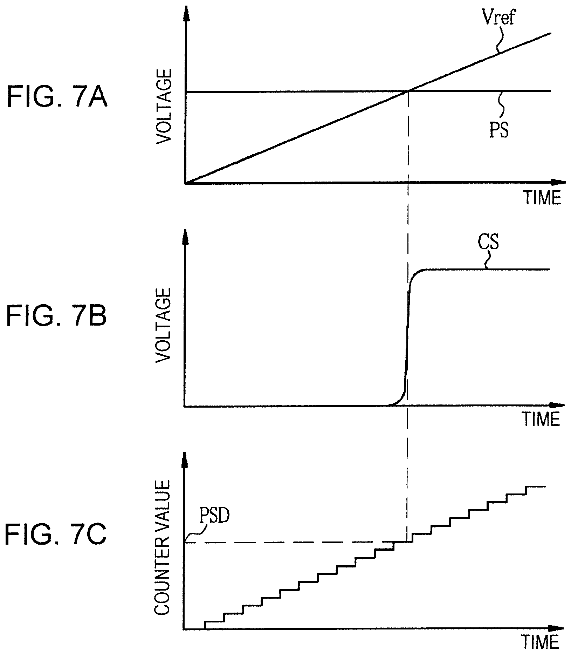

The reference signal generation unit 52c is connected in common to the third capacitors C3 of the comparators 52a and inputs a reference signal Vref to the third capacitors C3. Specifically, the reference signal generation unit 52c generates the reference signal Vref, which linearly increases as time passes as illustrated in Fig. 7A, on the basis of a clock signal input from the TG 55, and inputs the reference signal Vref to the third capacitors C3.

Each of the comparators 52a compares pixel signals PS input to the first capacitor C1 and to the second capacitor C2 with the reference signal Vref and outputs a signal CS that indicates the magnitude relationship between the voltage values of the signals, as illustrated in Fig. 7B. Specifically, each of the comparators 52a compares, in a case where a pixel signal PS is input to one of the first capacitor C1 and the second capacitor C2, the pixel signal PS with the reference signal Vref and compares, in a case where pixel signals PS are respectively input to the first capacitor C1 and to the second capacitor C2, the sum of the pixel signals PS with the reference signal Vref.

The output signal CS is input to a corresponding one of the counters 52b. The counter 52b starts a count operation when the reference signal Vref starts increasing, as illustrated in Fig. 7C, on the basis of the clock signal input from the TG 55. When the voltage value of the pixel signal PS matches the voltage value of the reference signal Vref, and the output signal CS changes from a low level to a high level, the counter 52b stops the count operation. A count value at the time when the counter 52b stops the count operation corresponds to the pixel signal PS. The count value is a digital signal and is output to the line memory 53 from the column ADC circuit 52 as a digitized pixel signal PSD.

The line memory 53 collectively retains the pixel signals PSD for one row digitized by the column ADC circuit 52. The column scanning circuit 54 scans the line memory 53 on the basis of a timing signal input from the TG 55 to thereby cause the pixel signals PSD to be sequentially output from an output terminal Vout. The pixel signals PSD for one frame output from the output terminal Vout correspond to the image capture signal described above.

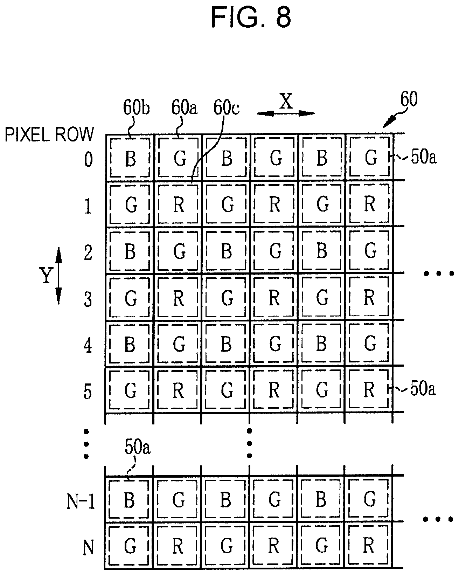

In Fig. 8, a color filter array 60 is provided on the light entrance side of the pixel array unit 50. The color filter array 60 includes green (G) filters 60a, blue (B) filters 60b, and red (R) filters 60c. On each of the pixels 50a, a corresponding one of these filters is arranged. The color arrangement of the color filter array 60 is based on the Bayer arrangement, in which the G filters 60a are arranged every other pixel in a checkered pattern, and the B filters 60b and the R filters 60c are arranged on the remaining pixels so that the B filters 60b and the R filters 60c are in square grid patterns respectively.

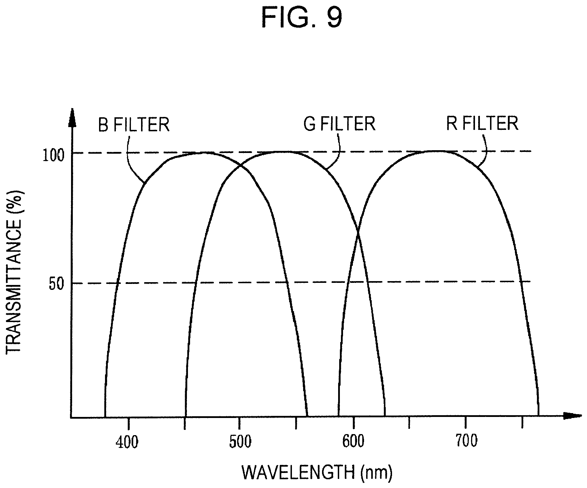

As illustrated in Fig. 9, the G filters 60a have a high transmittance in a wavelength range between 450 and 630 nm or so. The B filters 60b have a high transmittance in a wavelength range between 380 and 560 nm or so. The R filters 60c have a high transmittance in a wavelength range between 580 and 760 nm or so. Hereinafter, the pixels 50a on which the G filters 60a are arranged are referred to as G pixels, the pixels 50a on which the B filters 60b are arranged are referred to as B pixels, and the pixels 50a on which the R filters 60c are arranged are referred to as R pixels. Among these pixels, a group of pixels of highest sensitivity to different-absorption-wavelength light described below corresponds to the B pixels.

When the first illumination light is emitted, the first blue laser light and a shorter-wavelength component of the first fluorescence enter the B pixels, a main-wavelength component of the first fluorescence enters the G pixels, and a longer-wavelength component of the first fluorescence enters the R pixels. Similarly, when the second illumination light is emitted, the second blue laser light and a shorter-wavelength component of the second fluorescence enter the B pixels, a main-wavelength component of the second fluorescence enters the G pixels, and a longer-wavelength component of the second fluorescence enters the R pixels. Note that the emission intensity of the first blue laser light is larger than that of the first fluorescence, and the emission intensity of the second blue laser light is larger than that of the second fluorescence. Therefore, most of the light that enters the B pixels is formed of a component of the first blue laser light or a component of the second blue laser light.

As described above, the image sensor 39 is a single-panel-type color image sensor, and therefore, the image capture signal is divided into G, B, and R pixel signals.

As a signal read method, a "sequential read method", a "skip read method", and a "pixel addition read method" can be performed. In the sequential read method, the row scanning circuit 51 sequentially selects each of the row selection lines LS and supplies the row selection signal S1. As a result, signal reading is performed for the pixel rows from the first pixel row "0" to the last pixel row "N" one by one sequentially. In this sequential reading, when signal reading is performed for the pixel rows in the first pixel row group, the first switches SW1 are turned on and the second switches SW2 are turned off. When signal reading is performed for the pixel rows in the second pixel row group, the second switches SW2 are turned on and the first switches SW1 are turned off.

In the skip read method, the row scanning circuit 51 sequentially selects the row selection lines LS in the first pixel row group, which is part of all of the pixel rows, and supplies the row selection signal S1. As a result, signal reading is performed only for the pixel rows in the first pixel row group one by one sequentially. When this skip reading is performed, the first switches SW1 are turned on and the second switches SW2 are turned off.

In the pixel addition read method, the row scanning circuit 51 sequentially selects the row selection lines LS of each set of two pixel rows that are spaced apart from each other by one pixel row in the column direction and supplies the row selection signal S1 to the two row selection lines LS simultaneously. As a result, combinations of two pixel rows, namely, pixel rows "0" and "2", "1" and "3", "4" and "6", "5" and "7", and so on are sequentially selected and pixel addition reading is performed. That is, addition reading is performed for each set of each pixel 50a in each pixel row in the first pixel row group and a corresponding pixel 50a in a corresponding pixel row in the second pixel row group, the set of pixels 50a having filters of the same color being arranged thereon. When this pixel addition reading is performed, both the first switches SW1 and the second switches SW2 are turned on, and a pixel signal PS input to each of the first capacitors C1 and a pixel signal PS input to each of the second capacitors C2 are added together.

As a reset method, a "sequential reset method", a "collective reset method", and a "partial reset method" can be performed. In the sequential reset method, the row scanning circuit 51 sequentially selects each of the row reset lines LR and supplies the reset signal S2. As a result, the pixel rows are reset from the first pixel row "0" to the last pixel row "N" one by one sequentially in the sequential reset method.

In the collective reset method, the row scanning circuit 51 selects all of the row reset lines LR and supplies the reset signal S2 to all of the row reset lines LR collectively. As a result, all of the pixel rows in the pixel array unit 50 are simultaneously reset at once.

In the partial reset method, the row scanning circuit 51 supplies the reset signal S2 to the row reset lines LR of the pixel rows in the first pixel row group among all of the pixel rows collectively. As a result, half of the pixel rows in the pixel array unit 50 are simultaneously reset at once.

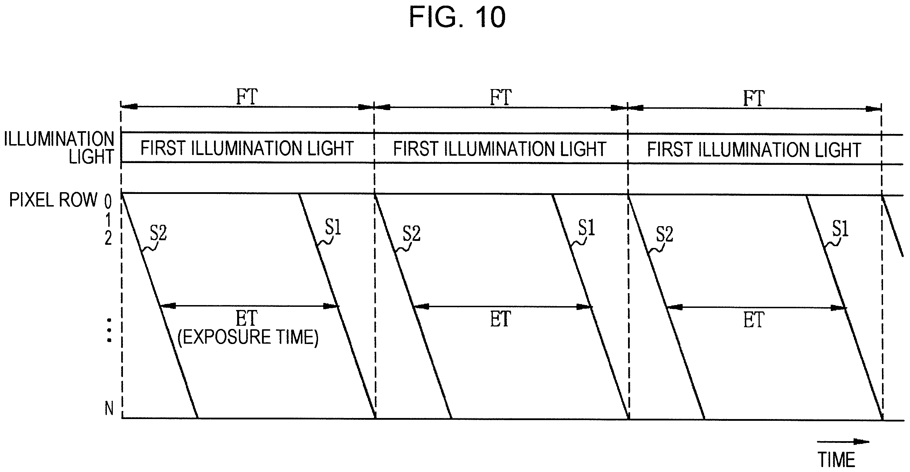

Now, control performed by the controller 41 in accordance with the observation mode is described. As illustrated in Fig. 10, in the normal observation mode, the controller 41 controls the light source controller 31 to turn on the first LD 30a, thereby causing the first illumination light to be emitted through a corresponding one of the illumination windows 23 of the endoscope 11. In a state where the first illumination light is being emitted, the controller 41 controls and drives the image sensor 39 in accordance with the rolling shutter method.

Specifically, the controller 41 first resets the pixel rows from the first pixel row "0" to the last pixel row "N" one by one sequentially in accordance with the sequential reset method. After an exposure time ET has passed since the start of the sequential resetting, the controller 41 performs signal reading for the pixel rows from the first pixel row "0" to the last pixel row "N" one by one sequentially in accordance with the sequential read method. As a result, an image capture signal for one frame is output from the image sensor 39. This driving in accordance with the rolling shutter method is repeatedly performed during the normal observation mode, and an image capture signal for one frame is obtained for each one-frame time FT (1/60 seconds, for example).

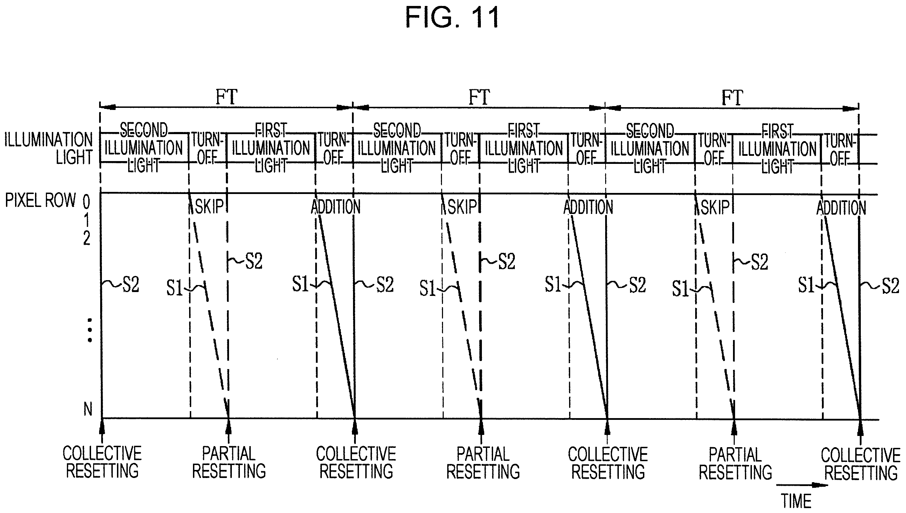

When the mode switching switch 22b is operated during the normal observation mode and the controller 41 receives a mode switching operation signal for giving an instruction for switching from the normal observation mode to the special observation mode, the controller 41 controls the light source controller 31 to turn on the first LD 30a and the second LD 30b alternately while interposing a turn-off period, thereby causing the first illumination light and the second illumination light to be emitted through the respective illumination windows 23 of the endoscope 11 alternately while interposing the turn-off period, as illustrated in Fig. 11.

Specifically, the controller 41 first causes the second illumination light to be emitted through a corresponding one of the illumination windows 23 of the endoscope 11, and resets all of the pixel rows simultaneously in accordance with the collective reset method. After a time equal to half the exposure time ET described above (ET/2) has passed since the collective resetting was performed, the controller 41 stops emission of the second illumination light and initiates a turn-off state. During this turn-off period, signal reading is performed sequentially for the pixel rows in the first pixel row group in accordance with the above-described skip read method.

Thereafter, the controller 41 performs resetting only for the first pixel row group in accordance with the partial reset method and causes the first illumination light to be emitted. After a time equal to half the exposure time ET (ET/2) has passed since the start of emission of the first illumination light, the controller 41 stops emission of the first illumination light and initiates a turn-off state. During this turn-off period, reading is performed while pixel addition for the first pixel row group and the second pixel row group is performed in accordance with the above-described pixel addition read method.

As a result, image capture signals for one frame are obtained. Among the image capture signals, an image capture signal (hereinafter referred to as a first image capture signal) that is read in accordance with the pixel addition read method after emission of the first illumination light is a combined signal formed of signals from the first pixel row group exposed only to the first illumination light and signals from the second pixel row group exposed to the first and second illumination light. The first image capture signal includes G, B, and R pixel signals, which are respectively referred to as G1 pixel signals, B1 pixel signals, and R1 pixel signals.

An image capture signal (hereinafter referred to as a second image capture signal) that is read in accordance with the skip read method after emission of the second illumination light corresponds to signals from the first pixel row group exposed only to the second illumination light. The second image capture signal includes G, B, and R pixel signals, which are respectively referred to as G2 pixel signals, B2 pixel signals, and R2 pixel signals.

As described above, in the special observation mode in this embodiment, signal reading is performed in accordance with the skip read method and the pixel addition read method, and therefore, the read time is equal to that in the conventional case of performing skip reading after emission of each type of illumination light. As a result, the special observation mode in this embodiment can be executed without a decrease in the frame rate from that in the normal observation mode.

As described above, the first and second image capture signals are input to the DSP 43 in the special observation mode. The DSP 43 performs a synchronization process and an interpolation process and generates one set of B1, G1, and R1 pixel signals and one set of B2, G2, and R2 pixel signals per pixel.

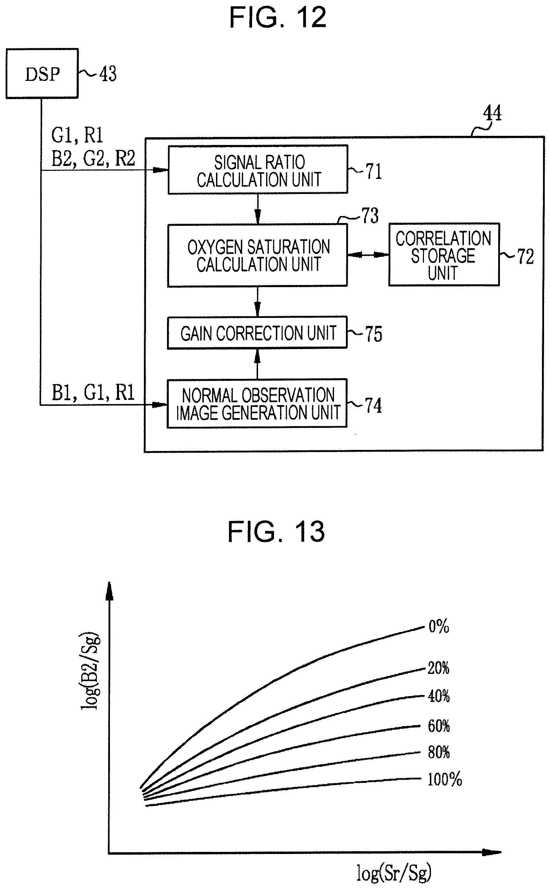

In Fig. 12, the image processor 44 of the processor apparatus 12 includes a signal ratio calculation unit 71, a correlation storage unit 72, an oxygen saturation calculation unit 73, a normal observation image generation unit 74, and a gain correction unit 75.

To the signal ratio calculation unit 71, the G1 pixel signals, the R1 pixel signals, the B2 pixel signals, the G2 pixel signals, and the R2 pixel signals in the first and second image capture signals input from the DSP 43 to the image processor 44 are input. The signal ratio calculation unit 71 first calculates for each pixel a green reference signal Sg and a red reference signal Sr on the basis of arithmetic expressions Sg = (G1 - G2)/2 and Sr = (R1 - R2)/2 respectively. The green reference signal Sg and the red reference signal Sr correspond to a G pixel signal value and an R pixel signal value in the case of emitting only the first illumination light. The signal ratio calculation unit 71 calculates a signal ratio B2/Sg between the B2 pixel signal and the green reference signal Sg and a signal ratio Sr/Sg between the red reference signal Sr and the green reference signal Sg.

The correlation storage unit 72 stores correlations between the signal ratios B2/Sg and Sr/Sg and oxygen saturation levels. These correlations are stored as a 2D table in which the isopleths of the oxygen saturation levels are defined in 2D space, as illustrated in Fig. 13. The positions and shapes of the isopleths relative to the signal ratio B2/Sg and the signal ratio Sr/Sg are obtained in advance by performing a physical simulation of light scattering, and the interval between the isopleths changes in accordance with the blood volume (the signal ratio Sr/Sg). Note that the correlations between the signal ratios B2/Sg and Sr/Sg and the oxygen saturation levels are stored on a logarithmic scale.

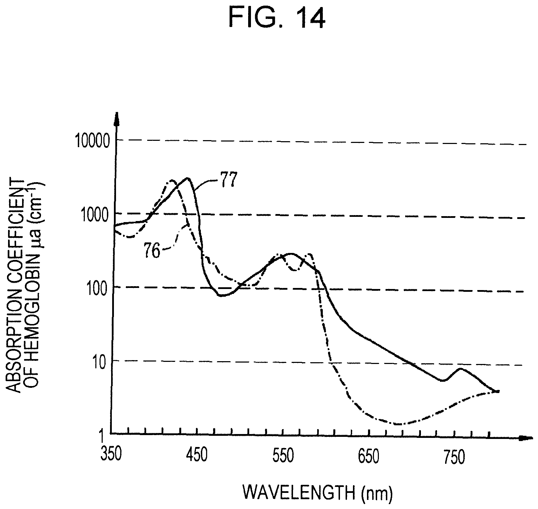

The above-described correlations closely relate to the absorption property of oxygenated hemoglobin (represented by the dotted-chain line 76) and the absorption property of reduced hemoglobin (represented by the solid line 77) illustrated in Fig. 14. An oxygen saturation level can be calculated by using light (different-absorption-wavelength light) having a certain wavelength, such as the second blue laser light having a center wavelength of 473 nm, which causes a large difference between the absorption coefficient of oxygenated hemoglobin and the absorption coefficient of reduced hemoglobin. However, the B2 pixel signals that mainly depend on the second blue laser light largely depend not only on the oxygen saturation level but also on the blood volume. In contrast, the red reference signals Sr that correspond to the R pixel signal values in the case of emitting only the first illumination light mainly depend on the blood volume.

Therefore, by using the values (the signal ratios B2/Sg and Sr/Sg) obtained by dividing each of the B2 pixel signals and each of the red reference signals Sr by each of the green reference signals Sg, which serves as a reference, the oxygen saturation level can be calculated with high accuracy while the dependence on the blood volume is reduced. A signal essential for calculating the oxygen saturation level is the B2 pixel signal, and therefore, the oxygen saturation level may be calculated only from the B2 pixel signal.

The oxygen saturation calculation unit 73 refers to the correlations stored on the correlation storage unit 72 and calculates for each pixel the oxygen saturation level that corresponds to the signal ratio B2/Sg and the signal ratio Sr/Sg calculated by the signal ratio calculation unit 71. The calculated value of the oxygen saturation level scarcely falls below 0% or exceeds 100%. In a case where the calculated value falls below 0%, the oxygen saturation level can be assumed to be 0%. In a case where the calculated value exceeds 100%, the oxygen saturation level can be assumed to be 100%.

The normal observation image generation unit 74 generates a normal observation image by using the B1, G1, and R1 pixel signals included in the first image capture signal. The first image capture signal is a combined signal formed of signals from the first pixel row group exposed only to the first illumination light and signals from the second pixel row group exposed to the first and second illumination light. Therefore, a normal observation image having a high brightness level and a good S/N value can be obtained by the normal observation image generation unit 74.

The gain correction unit 75 performs gain correction on each of the B1, G1, and R1 pixel signals that constitute each pixel of the normal observation image in accordance with the oxygen saturation level. For example, for a pixel for which the correction oxygen saturation level is 60% or more, the gain is set to "1" for all of the B1, G1, and R1 pixel signals. For a pixel for which the correction oxygen saturation level is less than 60%, the gain is set to a value smaller than "1" for the B1 pixel signal, and the gain is set to a value equal to or larger than "1" for the G1 and R1 pixel signals. Then, the B1, G1, and R1 pixel signals obtained after gain correction are used to generate an image. The normal observation image on which gain correction has been performed as described above is an oxygen saturation image. In this oxygen saturation image, a high oxygen area (an area having an oxygen saturation level between 60 and 100%) is colored similarly to the normal observation image, and the color of a low oxygen area (an area having an oxygen saturation level between 0 and 60%) is changed to blue.

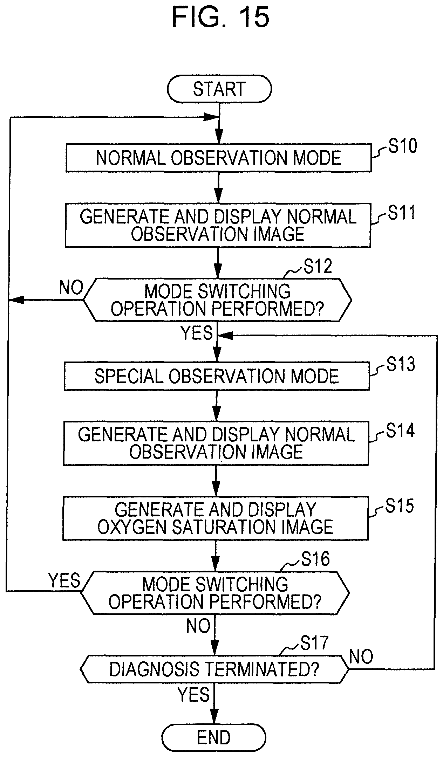

Now, an operation of the endoscope system 10 is described with reference to the flowchart in Fig. 15. First, an operator inserts the endoscope 11 into a living body and observes an observation region in the normal observation mode (step S10). In the normal observation mode, the image sensor 39 is driven in accordance with the rolling shutter method in a state where the first illumination light is being emitted to the observation region, and an image capture signal is read from the image sensor 39 for each one-frame time. On the basis of the image capture signal, a normal observation image is generated by the image processor 44 and is displayed on the monitor 14 (step S11). The display frame rate of the monitor 14 is equal to the frame rate of the image sensor 39, and the normal observation image displayed on the monitor 14 is refreshed for each one-frame time.

When the operator finds a region that is likely to be a lesion as a result of observation in the normal observation mode and operates the mode switching switch 22b to switch the observation mode (Yes in step S12), the observation mode transitions to the special observation mode (step S13).

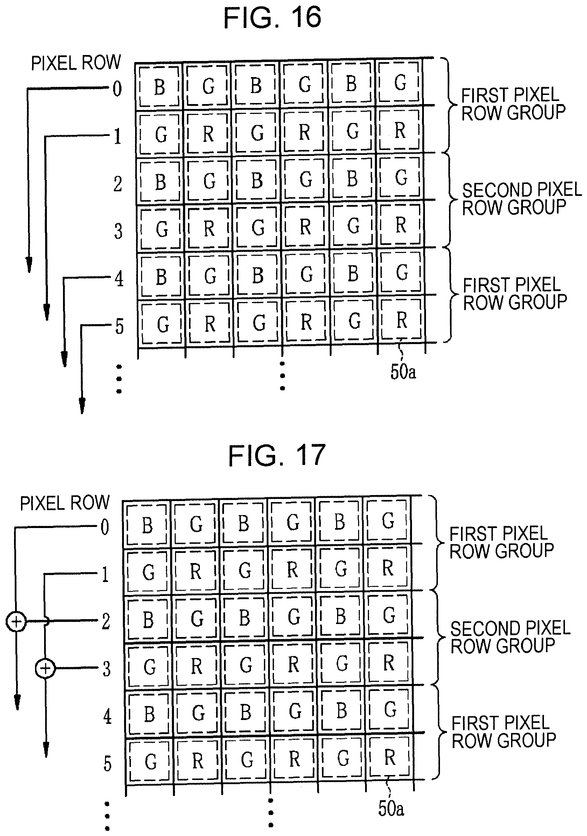

In the special observation mode, emission of the second illumination light to the observation region is started, collective resetting is performed for the image sensor 39, emission of the second illumination light is stopped, and reading is performed sequentially in sets of two pixel rows adjacent to each other in the column direction thereafter in the turn-off state in accordance with the skip read method while the immediately succeeding two pixel rows are skipped, as illustrated in Fig. 16. That is, signal reading for the first pixel row group is performed, and a second image capture signal is obtained.

Subsequently, emission of the first illumination light is started, only the first pixel row group is reset by partial resetting, emission of the first illumination light is stopped, and reading is performed while addition for each set of two pixels on which color filters of the same color are arranged is performed in the column direction thereafter in the turn-off state in accordance with the pixel addition read method, as illustrated in Fig. 17. That is, reading is performed while pixel addition for the first pixel row group and the second pixel row group is performed, and a first image capture signal is obtained.

As a result of the image capture method described above, first and second image capture signals are obtained for each one-frame time in the special observation mode. Each time first and second image capture signals are obtained, a normal observation image is generated by the image processor 44 on the basis of the first image capture signal and is displayed on the monitor 14 (step S14), and an oxygen saturation image is generated by the image processor 44 on the basis of the first and second image capture signals and is displayed on the monitor 14 (step S 15). The normal observation image and the oxygen saturation image are arranged side by side and displayed simultaneously on the screen of the monitor 14, for example.

Generation and display of a normal observation image and an oxygen saturation image are repeatedly performed until the operator operates the mode switching switch 22b again or performs an operation for terminating the diagnosis. If the mode switching switch 22b is operated (Yes in step S16), the observation mode returns to the normal observation mode (step S10), and a similar operation is performed. On the other hand, if the mode switching switch 22b is not operated and an operation for terminating the diagnosis is performed (Yes in step S 17), the operation of the endoscope system 10 is terminated.

### Second Embodiment

In the first embodiment described above, it is assumed that the first illumination light is light that includes the first blue laser light and that the second illumination light is light that includes the second blue laser light (different-absorption-wavelength light). In contrast, in the second embodiment, it is assumed that the first illumination light is light that includes the second blue laser light (different-absorption-wavelength light) and that the second illumination light is light that includes the first blue laser light.

This embodiment is based on a second image capture signal (G2 pixel signals, B2 pixel signals, and R2 pixel signals) read in accordance with the skip read method after emission of the second illumination light and a first image capture signal (G1 pixel signals, B1 pixel signals, and R1 pixel signals) read in accordance with the pixel addition read method after emission of the first illumination light. A B pixel signal in the case of emitting only the first illumination light that includes different-absorption-wavelength light is obtained by calculating an expression (B1 - B2)/2. A G pixel signal and an R pixel signal in the case of emitting only the second illumination light are obtained as a G2 pixel signal and an R2 pixel signal.

Therefore, in this embodiment, the signal ratio calculation unit 71 calculates a value of a signal ratio (B1 - B2)/(2 x G2) and a value of a signal ratio R2/G2. The oxygen saturation calculation unit 73 calculates the oxygen saturation level on the basis of the value of the signal ratio (B1 - B2)/(2 x G2) and that of the signal ratio R2/G2. The normal observation image generation unit 74 generates a normal observation image on the basis of the first image capture signal.

Also in this embodiment, a normal observation image having a high brightness level and a good S/N value can be obtained. In this embodiment, a signal corresponding to a B pixel signal in the case of emitting only light that includes different-absorption-wavelength light is obtained on the basis of the first image capture signal read in accordance with the pixel addition read method, which results in an increased S/N value. The configuration of this embodiment other than the above-described process is similar to that in the first embodiment.

Note that, in the above-described embodiments, an oxygen saturation image is generated by performing image processing on a normal observation image on the basis of the oxygen saturation level; however, an oxygen saturation image may be generated by representing information about the oxygen saturation level by an image.

In the above-described embodiments, collective resetting is performed upon the start of emission of the second illumination light, as illustrated in Fig. 11; however, collective resetting need not be performed and resetting may be performed in accordance with the sequential reset method during a turn-off period before the start of emission of the second illumination light.

In the above-described embodiments, partial resetting in which only the first pixel row group is reset is performed upon the start of emission of the first illumination light, as illustrated in Fig. 11; however, the partial resetting need not be performed. The image sensor 39 is of the CMOS type, and therefore, each pixel 50a retains a signal charge even after signal reading until resetting is performed. Accordingly, in the case where partial resetting is not performed, the first image capture signal is a signal obtained as a result of pixel addition performed for the first pixel row group exposed to the first and second illumination light and the second pixel row group exposed to the first and second illumination light, which results in a further increase in the brightness of the normal observation image.

In the above-described embodiments, a capacitance addition method is used as the pixel addition read method in which addition of pixel signals is performed by using the capacitors in each column ADC; however, a counter addition method in which addition is performed by using the counter in each column ADC or an FD addition method in which addition is performed by using a floating diffusion section may be used, for example.

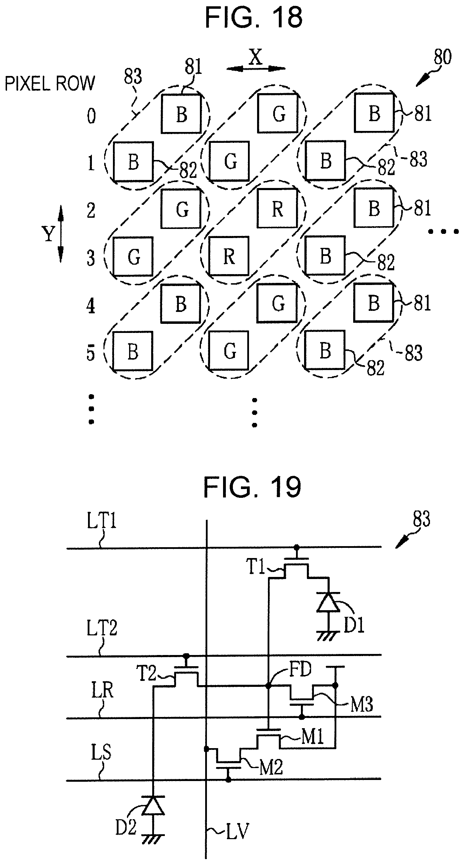

Examples of an image sensor that enables the FD addition method include an image sensor 80 of the CMOS type based on an EXR arrangement scheme illustrated in Fig. 18. The image sensor 80 includes a plurality of first pixels 81 arranged in even-numbered rows and a plurality of second pixels 82 arranged in odd-numbered rows. The first pixels 81 are arranged in a square grid pattern and constitute a first pixel row group. The second pixels 82 are arranged in a square grid pattern and constitute a second pixel row group. The first pixels 81 are arranged in the row direction while being spaced apart from each other by one pixel, the second pixels 82 are arranged in the row direction while being spaced apart from each other by one pixel, and the first pixels 81 are located at positions shifted by one pixel from the positions at which the second pixels 82 are located.

The first and second pixel row groups have a primary-color-type color filter array based on the Bayer arrangement. One pair of the first pixel 81 and the second pixel 82 adjacent to each other constitutes a pixel pair 83, on which color filters of the same color are arranged.

As illustrated in Fig. 19, the pixel pair 83 includes a first photodiode D1, a first transfer transistor T1, a second photodiode D2, a second transfer diode T2, a floating diffusion section FD, an amplifier transistor M1, a pixel selection transistor M2, and a reset transistor M3.

The first photodiode D1 and the first transfer transistor T1 are provided in the first pixel 81. The second photodiode D2 and the second transfer transistor T2 are provided in the second pixel 82. The floating diffusion section FD, the amplifier transistor M1, the pixel selection transistor M2, and the reset transistor M3 are shared by the first pixel 81 and the second pixel 82.

The first transfer transistor T1 is provided between the first photodiode D1 and the floating diffusion section FD and is controlled to be turned on and off by a first transfer control line LT1. When the first transfer transistor T1 is turned on, a signal charge accumulated in the first photodiode D1 is transferred to the floating diffusion section FD.

The second transfer transistor T2 is provided between the second photodiode D2 and the floating diffusion section FD and is controlled to be turned on and off by a second transfer control line LT2. When the second transfer transistor T2 is turned on, a signal charge accumulated in the second photodiode D2 is transferred to the floating diffusion section FD.

The amplifier transistor M1 converts the signal charge accumulated in the floating diffusion section FD into a voltage value (pixel signal). The pixel selection transistor M2 is controlled by a row selection line LS and causes the pixel signal generated by the amplifier transistor M1 to be output to a column signal line LV. The reset transistor M3 is controlled by a row reset line LR, and releases (resets) the signal charge accumulated in the floating diffusion section FD.

The first transfer control line LT1, the second transfer control line LT2, the row selection line LS, and the row reset line LR are connected in common to the pixel pairs 83 arranged in the row direction, and control signals are supplied therethrough from a row scanning circuit (not illustrated). The column signal line LV is connected in common to the pixel pairs 83 arranged in the column direction, and pixel signals are output therethrough to a column ADC circuit (not illustrated). The image sensor 80 further includes a line memory, a column scanning circuit, and a timing generator (which are not illustrated) as in the image sensor 39 of the first embodiment.

In the image sensor 80, pixel addition reading for the first pixel row group and the second pixel row group is performed by turning on both the first transfer transistor T1 and the second transfer transistor T2, transferring both the signal charge accumulated in the first photodiode D1 and that accumulated in the second photodiode D2 to the floating diffusion section FD, adding together the signal charges, and reading the result as a pixel signal.

Skip reading in which signal reading is performed only for the first pixel row group is performed by turning on only the first transfer transistor T1, transferring only the signal change accumulated in the first photodiode D1 to the floating diffusion section FD, and reading the signal charge as a pixel signal.

Sequential reading is performed by alternately turning on the first transistor T1 and the second transistor T2, alternately transferring the signal charge accumulated in the first photodiode D1 and that accumulated in the second photodiode D2 to the floating diffusion section FD, and reading the signal charge as a pixel signal.

Collective resetting is performed by turning on both the first transistor T1 and the second transistor T2, transferring both the signal charge accumulated in the first photodiode D1 and that accumulated in the second photodiode D2 to the floating diffusion section FD, and releasing the signal charges into a power supply line.

Partial resetting for resetting only the first pixel row group is performed by turning on only the first transfer transistor T1, transferring only the signal charge accumulated in the first photodiode D1 to the floating diffusion section FD, and releasing the signal charge into the power supply line.

Sequential resetting is performed by alternately turning on the first transistor T1 and the second transistor T2, alternately transferring the signal charge accumulated in the first photodiode D1 and that accumulated in the second photodiode D2 to the floating diffusion section FD, and releasing the signal charge into the power supply line. Operations performed by the image sensor 80 other than the above-described operations are similar to those performed by the image sensor 39 of the first embodiment.

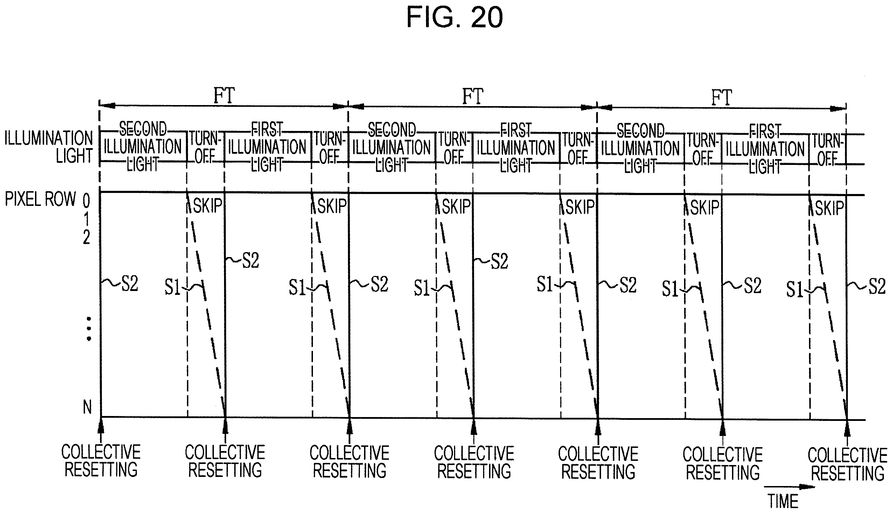

In the special observation mode in the above-described embodiments, the light source apparatus 13 and the image sensor 39 are driven in accordance with the image capture method illustrated in Fig. 11 (hereinafter referred to as a first image capture method). In addition to this image capture method, the light source apparatus 13 and the image sensor 39 may be driven in accordance with a conventional image capture method illustrated in Fig. 20 (hereinafter referred to as a second image capture method).

In the second image capture method, the first illumination light and the second illumination light are alternately emitted while a turn-off period is interposed, and signal reading is performed during the turn-off period in accordance with the skip read method. Upon the start of emission of each type of illumination light, all pixel rows are simultaneously reset at once in accordance with the collective reset method. In the signal reading, pixel skipping is performed by performing reading only for the first pixel row group described above in the pixel array unit 50, for example. The frame rate in the second image capture method is equal to that in the first image capture method.

In the second image capture method, a normal observation image is generated on the basis of an image capture signal read after emission of the first illumination light. An oxygen saturation image is generated on the basis of an image capture signal read after emission of the first illumination light and an image capture signal read after emission of the second illumination light. An oxygen saturation image can be generated by using only an image capture signal read after emission of the second illumination light. In the second image capture method, a normal observation image is generated on the basis of an image capture signal obtained from the first illumination light as in the case of a normal observation image in the normal observation mode, and therefore, the same white balance process, and so on performed by the DSP 43 in the normal observation mode can be performed.

In the first image capture method, the brightness and S/N of a normal observation image are improved; however, the exposure time is longer than that in the second image capture method, and blurring caused by the subject movement, the camera, and so on often occurs. Accordingly, switching between the first image capture method and the second image capture method may be performed in accordance with the brightness of the test body.

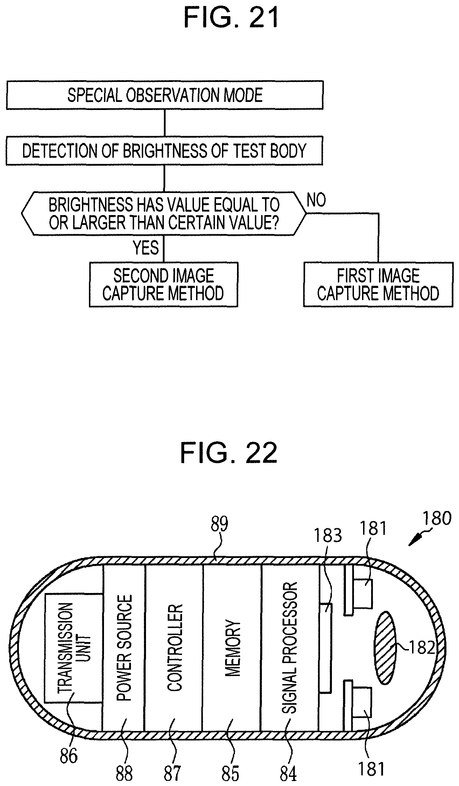

Specifically, after switching from the normal observation mode to the special observation mode, the brightness of the test body is detected, as illustrated in Fig. 21. The brightness of the test body is detected by the DSP 43 on the basis of the image capture signals. For example, the brightness of the test body is obtained by calculating the average brightness value from the image capture signals for one frame. That is, the DSP 43 corresponds to a brightness detection unit. In the brightness detection, the image capture signals obtained in accordance with the first image capture method or the image capture signals obtained in accordance with the second image capture method may be used.

After detection of the brightness of the test body, the second image capture method is selected in a case where the brightness has a value equal to or larger than a certain value, and the first image capture method is selected in a case where the brightness has a value smaller than the certain value. Note that the brightness of the test body may be calculated in the normal observation mode, and an image capture method may be selected on the basis of the brightness calculated in the normal observation mode upon switching to the special observation mode.

Alternatively, determination as to whether a gain of a certain value or more is necessary in the DSP 43 or the like because of a low S/N value of the image capture signal may be performed instead of determination of the brightness of the test body. In a case where a gain of a certain value or more is necessary, the first image capture method may be selected, and in a case where a gain of a certain value or more is not necessary, the second image capture method may be selected.

Also in the second image capture method, collective resetting need not be performed upon the start of emission of each type of illumination light, and resetting may be performed during the turn-off period in accordance with the sequential reset method.

In the above-described embodiments, the color filter array of the primary color type is used; however, this type of color filter array may be replaced and a complementary-color-type color filter array may be used.

In the above-described embodiments, the first laser light emitted from the first LD 30a and the second laser light emitted from the second LD 30b are emitted to the fluorescent body 36 to thereby generate the first and second illumination light; however, the first and second illumination light may be generated by using a white light source, such as a xenon lamp, and a wavelength separation filter as disclosed by Japanese Unexamined Patent Application Publication No. 2013-165776. Further, it is possible to generate the first and second illumination light by using LEDs (three types of LEDs that respectively emit R, G, and B light, for example) and a wavelength selection filter.

In the above-described embodiments, white light is used as the first illumination light and special light including light that has a high absorption coefficient for blood hemoglobin is used as the second illumination light to generate an oxygen saturation image as a special observation image; however, narrowband light (violet narrowband light having a center wavelength of 405 nm, for example) that has a high absorption coefficient for blood hemoglobin may be used as the second illumination light to generate, as a special observation image, a blood vessel enhancement observation image in which the blood vessels on the tissue surface are enhanced.

In the above-described embodiments, the light source apparatus and the processor apparatus are configured separately; however, the light source apparatus and the processor apparatus may be configured as a single apparatus.

The present invention is applicable to a capsule endoscope that captures images while passing through an alimentary canal and transfers the captured images to a recording apparatus. For example, a capsule endoscope 180 is constituted by an illumination unit 181, a lens 182, an image sensor 183, a signal processor 84, a memory 85, a transmission unit 86, a controller 87, a power source 88, and a capsule housing 89 that houses these components, as illustrated in Fig. 22.

The illumination unit 181 includes an LED and a wavelength selection filter and emits the above-described first and second illumination light to a test body. The image sensor 183 is of the CMOS type that captures, via the lens 182, images of reflection light from the test body illuminated with the first and second illumination light and outputs the above-described first and second image capture signals. The signal processor 84 performs signal processing, which is performed by the DSP 43 and the image processor 44 in the above-described embodiment, on the first and second image capture signals and generates a normal observation image and an oxygen saturation image. The memory 85 stores the images. The transmission unit 86 wirelessly transmits the images stored in the memory 85 to an external recording apparatus (not illustrated). The controller 87 controls the components.

Note that the first and second image capture signals may be transmitted from the transmission unit 86 to an external apparatus (not illustrated), and the external apparatus may generate a normal observation image and an oxygen saturation image.

The present invention is applicable to a fiberscope in which reflection light from an observation region resulting from illumination light is guided by an image guide and to an endoscope system using an ultrasonic endoscope having a distal end into which an image sensor and an ultrasonic transducer are built.

### Reference Signs List

- 10: endoscope system
- 11: endoscope
- 12: processor apparatus
- 13: light source apparatus
- 30a: first laser diode
- 30b: second laser diode
- 35: light guide
- 36: fluorescent body
- 39: image sensor
- 41: controller
- 50: pixel array unit
- 50a: pixel
- 80: image sensor
- 81: first pixel
- 82: second pixel
- 83: pixel pair

## Claims

1. An endoscope system comprising:
an illumination unit that emits to a test body first illumination light and second illumination light having different spectral properties;
an endoscope including an image sensor of CMOS type that captures an image of the test body illuminated by the illumination unit by using a plurality of pixel rows arranged in a column direction;
a controller that causes the illumination unit and the image sensor to perform a first image capture method in which the second illumination light is emitted from the illumination unit, the illumination unit is thereafter turned off, signal reading is performed for pixel rows among the plurality of pixel rows in accordance with a skip read method, the first illumination light is emitted from the illumination unit, the illumination unit is thereafter turned off, and signal reading is performed for the plurality of pixel rows in the column direction in accordance with a pixel addition read method; and
an image processor that generates a special observation image on the basis of a second image capture signal read in accordance with the skip read method and a first image capture signal read in accordance with the pixel addition read method, and generates a normal observation image on the basis of the first image capture signal.

2. The endoscope system according to claim 1, wherein
the controller controls the image sensor to reset only the pixel rows for which signal reading has been performed in accordance with the skip read method after signal reading in accordance with the skip read method has been performed.

3. The endoscope system according to claim 1 or 2, wherein
the image sensor includes a color filter based on a Bayer arrangement,
in the skip read method, reading is performed sequentially in sets of two pixel rows adjacent to each other in the column direction while immediately succeeding two pixel rows are skipped, and
in the pixel addition read method, reading is performed while addition for each set of two pixels on which color filters of an identical color are arranged is performed in the column direction.

4. The endoscope system according to claim I or 2, wherein
in the image sensor, paired pixels adjacent to each other selectively share a floating diffusion section,
in the skip read method, only a signal charge accumulated in one of the paired pixels is read via the floating diffusion section, and
in the pixel addition read method, signal charges accumulated in the paired pixels are added together by the floating diffusion section and read.

5. The endoscope system according to any one of claims 1 to 4, wherein
the first illumination light or the second illumination light includes different-absorption-wavelength light that has different absorption coefficients for oxygenated hemoglobin and reduced hemoglobin, and
the image processor generates an oxygen saturation image that includes information about an oxygen saturation level as the special observation image.

6. The endoscope system according to claim 5, wherein
the image processor generates the oxygen saturation image by calculating the oxygen saturation level on the basis of the first image capture signal and the second image capture signal and performing image processing on the normal observation image on the basis of the oxygen saturation level.

7. The endoscope system according to any one of claims 1 to 6, wherein
the controller enables the illumination unit and the image sensor to perform a second image capture method in which the first illumination light is emitted from the illumination light, the illumination unit is thereafter turned off, signal reading is performed in accordance with the skip read method, the second illumination light is emitted from the illumination unit, the illumination unit is thereafter turned off, and signal reading is performed in accordance with the skip read method.

8. The endoscope system according to claim 7, further comprising:
a brightness detection unit that detects brightness of the test body, wherein
the controller causes the first image capture method to be performed in a case where the brightness has a value smaller than a certain value, and causes the second image capture method to be performed in a case where the brightness has a value equal to or larger than the certain value.

9. A processor apparatus for an endoscope system, the endoscope system including
an illumination unit that emits to a test body first illumination light and second illumination light having different spectral properties, and
an endoscope including an image sensor of CMOS type that captures an image of the test body illuminated by the illumination unit by using a plurality of pixel rows arranged in a column direction,
the processor apparatus comprising:
a controller that causes the illumination unit and the image sensor to perform an image capture method in which the second illumination light is emitted from the illumination unit, the illumination unit is thereafter turned off, signal reading is performed for pixel rows among the plurality of pixel rows in accordance with a skip read method, the first illumination light is emitted from the illumination unit, the illumination unit is thereafter turned off, and signal reading is performed for the plurality of pixel rows in the column direction in accordance with a pixel addition read method; and
an image processor that generates a special observation image on the basis of a second image capture signal read in accordance with the skip read method and a first image capture signal read in accordance with the pixel addition read method, and generates a normal observation image on the basis of the first image capture signal.

10. A method for operating an endoscope system, the endoscope system including
an illumination unit that emits to a test body first illumination light and second illumination light having different spectral properties, and
an endoscope including an image sensor of CMOS type that captures an image of the test body illuminated by the illumination unit by using a plurality of pixel rows arranged in a column direction,
the method comprising the steps of:
causing, by a controller, the illumination unit and the image sensor to perform an image capture method in which the second illumination light is emitted from the illumination unit, the illumination unit is thereafter turned off, signal reading is performed for pixel rows among the plurality of pixel rows in accordance with a skip read method, the first illumination light is emitted from the illumination unit, the illumination unit is thereafter turned off, and signal reading is performed for the plurality of pixel rows in the column direction in accordance with a pixel addition read method; and
generating, by an image processor, a special observation image on the basis of a second image capture signal read in accordance with the skip read method and a first image capture signal read in accordance with the pixel addition read method, and generating a normal observation image on the basis of the first image capture signal.
